Europäisches Patentamt

European Patent Office

Office européen des brevets

(19)

(11) Veröffentlichungsnummer: **0 215 423 B1**

(12) **EUROPÄISCHE PATENTSCHRIFT**

(45) Veröffentlichungstag der Patentschrift: 08.05.91

(51) Int. Cl.⁵: **A61K 31/19**, A61K 47/00

(21) Anmeldenummer: 86112457.6

(22) Anmeldetag: 09.09.86

Die Akte enthält technische Angaben, die nach dem Eingang der Anmeldung eingereicht wurden und die nicht in dieser Patentschrift enthalten sind.

(54) **Transdermal resorbierbare, wasserhaltige Zubereitungen von Arylpropionsäurederivaten und Verfahren zur Herstellung derselben.**

(30) Priorität: 12.09.85 DE 3532562

(43) Veröffentlichungstag der Anmeldung:
25.03.87 Patentblatt 87/13

(45) Bekanntmachung des Hinweises auf die Patenterteilung:
08.05.91 Patentblatt 91/19

(84) Benannte Vertragsstaaten:
AT BE CH DE FR GB IT LI LU NL SE

(56) Entgegenhaltungen:
EP-A- 0 072 462   EP-A- 0 178 436
DE-A- 1 617 480   DE-A- 2 708 152
US-A- 3 740 421   US-A- 4 511 563

ROTE LISTE, Editio Cantor, Aulendorf/Württ., DE

CHEMICAL ABSTRACTS, Band 104, Nr. 20, 19. Mai 1986, Seite 392, Zusammenfassung Nr. 174678h, Columbus, Ohio, US; & JP-A-61 27 920 (AKIYOSHI, MASATAKA; NAKANISHI, MICHIO) 07-02-1986

(73) Patentinhaber: Dolorgiet GmbH & Co. KG
Otto-von-Guericke-Str. 1
W-5205 St. Augustin 3(DE)

(72) Erfinder: Löhner, Manfred
Blücherstrasse 47
W-5300 Bonn 1(DE)
Erfinder: Wagener, Hans Henrich
Breslauer Strasse 76
W-5309 Meckenheim(DE)

(74) Vertreter: Werner, Hans-Karsten, Dr. et al
Deichmannhaus am Hauptbahnhof
W-5000 Köln 1(DE)

## Beschreibung

Gegenstand der vorliegenden Erfindung sind transdermal resorbierbare, wasserhaltige Zubereitungen von Arylpropionsäuren sowie Verfahren zur Herstellung derselben, die in Form von Einreibungen, vorzugsweise aber in Form von Gelen vorliegen.

Arylpropionsäuren wie z.B. Flurbiprofen = 2-(2-Fluorbiphenyl-4-yl)-propionsäure, Ibuprofen = 2-(4-Isobutylphenyl)-propionsäure, Ketoprofen 2-(3-Benzoylphenyl)-propionsäure oder Naproxen = (+)-2-(6-Methoxy-2-naphthyl)-propionsäure sind aus Martindale, The Extra Pharmacopoeia, 28. Auflage, 1982, Seiten 255, 256, 261 und 264 als Arzneistoffe mit antiinflammatorischen und analgetischen Eigenschaften bekannt. Sie werden zur Behandlung von rheumatoider Arthritis oder anderen entzündlichen Gelenkerkrankungen, Weichteilrheumatismus und Gicht eingesetzt.

Die Verabreichung der Arylpropionsäuren erfolgt vornehmlich in Form von Tabletten und Dragees. Die orale Anwendung verursacht jedoch auch nachteilige Wirkungen wie gastrointestinale Beschwerden, Schwindel, Übelkeit und Kopfschmerzen. Bei Patienten mit Ulcus ventriculi und duodeni ist eine orale Behandlung mit diesen Arzneimitteln ausgeschlossen.

Ein transdermal zur Absorption gebrachter Wirkstoff erreicht das Zielorgan unmittelbar unter Umgehung des bei oraler Verabreichung unumgänglichen First-pass-Metabolismus, d.h. der ersten Verstoffwechslung in der Leber.

Hierdurch wird es möglich, die für den pharmakodynamischen Effekt erforderliche Wirkstoffdosis und damit auch die bei oraler Anwendung auftretenden Nachteile zu vermindern.

Aus diesem Grunde sind bereits Versuche unternommen worden, Arylpropionsäuren enthaltende Arzneimittel zur transdermalen Anwendung herzustellen. Aus der DE-PS 32 05 504 bzw. EP-OS 0 087 062 ist eine Zusammensetzung mit Ibuprofen in Form einer Creme bekannt, die auch schon als Handelsprodukt zur Anwendung kommt.

Aus Boll. Chim. Farm., 119 (1980), Seite 738, sind dermatologische Formulierungen bekannt,die mit verschiedenen Wirkstoffen bezüglich der in-vitro-Freisetzung (Diffusionsgeschwindigkeit) untersucht wurden. Daraus geht hervor, daß wasserhaltige Zubereitungen höhere Freisetzungsraten aufweisen als wasserfreie. Die Versuche wurden einheitlich mit 2,5 Gew.-% Wirkstoff durchgeführt. Höhere Konzentrationen wurden nicht untersucht. Es fehlen jegliche Angaben, die auf eine ausreichende therapeutische Wirksamkeit der so erhaltenen Produkte schließen lassen.

In der EP-OS 0 127 840 sind Zusammensetzungen mit einigen Arylessig- und Arylpropionsäuren beschrieben, die hinsichtlich der Rezeptur dem Gel der obigen Publikation entsprechen. Auf Wirksamkeit wurden nur Zubereitungen mit dem Arylessigsäurederivat Ibufenac unterschiedlicher Konzentration geprüft.

Aus der DE-OS 31 19 017 sind Gele mit den Arylpropionsäuren Ketoprofen und Flurbiprofen bekannt, die in 1%iger Zubereitung auf perkutane Absorption an Menschen und auf entzündungshemmende Aktivität an Ratten untersucht wurden. Diese Gele enthalten außer dem als Gelgrundlage geläufigen Carboxyvinylpolymer zusätzliche Hilfsstoffe.

Die DE-OS 33 36 047 und EP-OS 0 072 462 beschreiben neben anderen topischen Darreichungsformen Gele mit verschiedenen Arylessig- und Arylpropionsäuren, von denen lediglich Flurbiprofen in ebenfalls 1%iger Zubereitung auf entzündungshemmende Wirkung an Tieren geprüft wurde. Auch diese Gele enthalten neben dem üblichen Carboxyvinylpolymer weitere Hilfsstoffe als Lösungsvermittler.

Obwohl in einigen der vorgenannten Publikationen zur Herstellung der Gele unterschiedliche gelbildende Mittel aufgezählt werden, wurden in den Ausführungsbeispielen ausschließlich saure Gruppen enthaltende Carboxyvinylpolymere (Carbopol® und Hiviswako®) als Gelierungsmittel eingesetzt. Da die Gelbildung jedoch nur in neutralem bzw. alkalischem Milieu erfolgt, mußte außer den Carboxyvinylpolymeren auch der saure Wirkstoff neutralisiert werden. Zur Neutralisation werden vorzugsweise wasserlösliche organische Amine eingesetzt, in wenigen Fällen auch Alkalibasen. Das führt zwangsläufig zur Bildung von Salzen der Wirkstoffe.

Ein lokal anwendbares Arzneimittel kann aber nur gut und rasch wirken, wenn der Wirkstoff durch die Haut penetriert. Vorzugsweise sollte deshalb der Wirkstoff gelöst sein. Die bereits oben erwähnte Bildung von Salzen läßt üblicherweise auch eine Verbesserung der Löslichkeit der Wirkstoffe erwarten.

Keines der Ausführungsbeispiele beschreibt Gele, die mehr als 3 % Arylpropionsäuren als Wirkstoff enthalten. Selbst mit zusätzlichen Solubilisierungs- oder oberflächenaktiven Mitteln unterschiedlichster Art wird diese Wirkstoffkonzentration nicht überschritten. Daraus kann gefolgert werden, daß eine übliche Carboxyvinylpolymer enthaltende Gelgrundlage ungeeignet ist zur Bereitung höherprozentiger Gele mit Arylpropionsäuren.

Arylpropionsäuren sind zwar in einigen physiologisch verträglichen Trägern löslich, fallen aber, wenn sie als Säure vorliegen, bei Zugabe von wenig Wasser sofort aus. Damit werden die Arylpropionsäuren,

2

selbst wenn sie in der wasserhaltigen Gelmasse verteilt bleiben, auf der Haut einer schnellen Resorption entzogen.

Aus H.P. Fiedler, Lexikon der Hilfsstoffe für Pharmazie, Kosmetik und angrenzende Gebiete, Editio Cantor Aulendorf, 1981, Seite 720 bzw. The Merck Index, 10th Edition, 1983, No. 7432 sind Polyoxyethylen-polyoxypropylen-copolymere (Pluronic® bzw. Poloxamer®) als Trägerstoffe bekannt, von denen einige Typen unter entsprechenden Bedingungen auch zur Gelbildung befähigt sind.

Aus der US-PS 4,511,563 ist bekannt, klare, analgetische Gele mit verminderter Klebrigkeit herzustellen aus neutralen oder mit Basen neutralisierten Analgetika wie Triethanolamin-salicylat, Kampher, Phenol-Kampher, Menthol etc., indem 10 bis 40 Teile eines nichtionischen Netzmittels wie Polyoxyethylen-polyoxypropylen-copolymere mit Wasser und 5 bis 40 Teilen Glycerin zu Gelen verarbeitet werden. Glycerin ist wesentlicher Bestandteil der Gele, da es die Klebrigkeit herabsetzt und die Gelbildung fördert.

Die Erfindung hat sich die Aufgabe gestellt, transdermal resorbierbare, wasserhaltige Zubereitungen von Arylpropionsäuren zu entwickeln, welche vorzugsweise auch als Gele vorliegen und zumindest eine ausreichende Resorption bei guter Verträglichkeit gewährleisten, ohne daß eine Neutralisation durch Basen erforderlich ist.

Überraschenderweise wurde gefunden, daß sogar wesentlich verbesserte und beschleunigte Resorption der Wirkstoffe erreicht werden kann, wenn die Zubereitungen neben 1 bis 15 Gew.-% Wirkstoff, 10 bis 40 Gew.-% Polyoxyethylen-polyoxypropylen-copolymere, 10 bis 50 Gew.-% eines oder mehrerer physiologisch verträglicher organischer Lösungsmittel, gegebenenfalls bis zu 1 Gew.-% Konservierungsstoffe, Farbstoffe und/oder Parfüme und mindestens 10 Gew.-% Wasser enthalten.

Geeignete Polyoxyethylen-polyoxypropylen-copolymere weisen relative Molmassen von 1000 bis 13000 auf. Sofern die relative Molmasse über 6000 liegt, ist es möglich, die erfindungsgemäßen Zubereitungen auch als Gele herzustellen. Dazu ist es nötig, daß neben 1 bis 10 Gew.-% Wirkstoff, 15 bis 32 Gew.-% Polyoxyethylen-polyoxypropylen-copolymere, 17 bis 44 Gew.-% eines oder mehrerer physiologisch verträglicher organischer Lösungsmittel und gegebenenfalls bis zu 1 Gew.-% Konservierungsstoffe, Farbstoffe und/oder Parfüme enthalten sind. Der Wassergehalt dieser Gele liegt meist über 30%.

Die Polyoxyethylen-polyoxypropylen-copolymere sind handelsüblich und werden beispielsweise unter den Bezeichnungen Pluronic® und Poloxamer® angeboten. Sie sind physiologisch verträglich und deshalb als Hilfsmittel zur Herstellung transdermal applizierbarer Arzneimittel geeignet.

Als physiologisch verträgliche organische Lösungsmittel können beispielsweise verwendet werden Dimethylsulfoxid oder ein-bis dreiwertige, geradkettige, verzweigte oder cyclische Alkohole mit 2 bis 6 Kohlenstoffatomen, in deren Kohlenstoffgerüst auch bis zu 2 Sauerstoffatome eingebaut sein können, oder Polyoxyalkylen-fettalkoholether sowie Gemische derselben. Bewährt haben sich beispielsweise Ethanol, Isopropanol, 1,2-Propandiol, Glycerin, Isosorbid, Dimethylisosorbid, Polyoxyethylen(4)-laurylether, Polyoxypropylen-(15)-stearylether sowie Dimethylsulfoxid.

Das Verfahren zur Herstellung der erfindungsgemäßen transdermal resorbierbaren, wasserhaltigen Zubereitungen von Arylpropionsäurederivaten erfolgt dadurch, daß die Komponenten gegebenenfalls unter Überdruck unter Rühren auf 40 bis 90 °C erwärmt und wieder abgekühlt werden. Der Überdruck ist insbesondere dann erforderlich, wenn die verwendeten organischen Lösungsmittel unter den Reaktionsbedingungen bereits verdampfen würden. Ein Überdruck von 0,2 bis 1 bar reicht völlig aus. Vorzugsweise wird im Bereich zwischen 0,2 und 0,5 bar gearbeitet.

Sofern Zubereitungen als Gele vorliegen sollen, ist es erforderlich, die relative Molmasse der Polyoxyethylen-polyoxypropylen-copolymere bei mindestens 6000 zu wählen. Handelsprodukte mit relativen Molmassen über 13000 scheinen bisher nicht im Handel zu sein.

Weiterhin ist bei der Herstellung von Gelen zu beachten, daß die Mengenverhältnisse zwischen Wirkstoff, Copolymer und organischem Lösungsmittel im Bereich von 1 bis 10 Gew.-% Wirkstoff, 15 bis 32 Gew.-% Polyoxyethylen-polyoxypropylen-copolymeren und 17 bis 44 Gew.-% des organischen Lösungsmittels gewählt werden.

Sofern eine in diesen Bereich fallende Mischung nicht zu einem Gel erstarren sollte, ist durch einfache Variation der Mengenverhältnisse dennoch das gewünschte Ziel erreichbar.

Die Auflösung der Wirkstoffe in dem Gemisch erfolgt vorzugsweise unter Rühren. Auch das Abkühlen auf Raumtemperatur kann unter Rühren erfolgen.

In den nachfolgenden Beispielen sind typische erfindungsgemäße Zubereitungen, insbesondere solche, die als Gele anfallen, näher erläutert.

**Beispiel 1**

```
 20,0 kg Ibuprofen
 64,0 kg Isopropanol
 40,0 kg Dimethylisosorbid
 64,0 kg Polyoxyethylen-polyoxypropylen-copolymer 12500
          (Pluronic® F 127, Wyandotte Chemicals Corp.)
211,7 kg Wasser
```

werden 10 Minuten lang intensiv gemischt. Die Mischung wird anschließend unter Rühren und leichtem Druck (0,3 bar) auf 85 °C erhitzt und 15 Minuten bei dieser Temperatur belassen. Danach wird auf 65 °C abgekühlt und mit Aromastoffen (0,1 kg Lavendelöl und 0,2 kg Neroliöl) versetzt. Die Lösung wird bei laufendem Rührwerk auf Raumtemperatur abgekühlt. Während der Abkühlphase geliert die Lösung ab einer Temperatur von ca. 45 °C zu einem klaren Gel. Das Gel enthält 5 % Ibuprofen.

**Beispiel 2**

```
 7,500 g Ibuprofen
15,925 g Isopropanol
10,000 g Dimethylisosorbid
16,000 g Polyoxyethylen-polyoxypropylen-copolymer 12500
          (Pluronic® F 127, Wyandotte Chemicals Corp.)
50,500 g Wasser
 0,025 g Lavendelöl
 0,050 g Neroliöl
```

werden wie in Beispiel 1 beschrieben verarbeitet. Das Gel enthält 7,5 % Ibuprofen.

**Beispiel 3**

```
10,000 g Ibuprofen
15,925 g Isopropanol
12,000 g Dimethylisosorbid
18,000 g Polyoxyethylen-polyoxypropylen-copolymer 12500
          (Pluronic® F 127, Wyandotte Chemicals Corp.)
44,000 g Wasser
 0,025 g Lavendelöl
 0,050 g Neroliöl
```

werden wie in Beispiel 1 beschrieben verarbeitet. Das Gel enthält 10 % Ibuprofen.

**Beispiel 4**

```
 5,00 g Ibuprofen
16,00 g Isopropanol
10,00 g Glycerin
16,00 g Polyoxyethylen-polyoxypropylen-copolymer 12500
        (Pluronic® F 127, Wyandotte Chemicals Corp.)
53,00 g Wasser
```

werden 10 Minuten lang intensiv gemischt.

Die Mischung wird anschließend unter Rühren und leichtem Druck (0,3 bar) auf 85 °C erhitzt und 15 Minuten bei dieser Temperatur belassen. Die Lösung wird bei laufendem Rührwerk auf Raumtemperatur abgekühlt. Während der Abkühlphase geliert die Lösung ab einer Temperatur von ca. 45 °C zu einem klaren Gel. Das Gel enthält 5 % Ibuprofen.

**Beispiel 5**

```
 5,00 g Ibuprofen
16,00 g Isopropanol
10,00 g Dimethylsulfoxid
16,00 g Polyoxyethylen-polyoxypropylen-copolymer 12500
        (Pluronic® F 127, Wyandotte Chemicals Corp.)
53,00 g Wasser
```

werden wie in Beispiel 4 beschrieben verarbeitet. Das Gel enthält 5 % Ibuprofen.

**Beispiel 6**

```
 5,00 g Ibuprofen
17,90 g Isopropanol
17,90 g Polyoxyethylen-polyoxypropylen-copolymer 12500
        (Pluronic® F 127, Wyandotte Chemicals Corp.)
59,20 g Wasser
```

werden wie in Beispiel 4 beschrieben verarbeitet. Das Gel enthält 5 % Ibuprofen.

**Beispiel 7**

```
 5,00 g Ibuprofen
31,00 g Dimethylisosorbid
15,50 g Polyoxyethylen-polyoxypropylen-copolymer 12500
        (Pluronic® F 127, Wyandotte Chemicals Corp.)
48,50 g Wasser
```

werden wie in Beispiel 4 beschrieben verarbeitet. Das Gel enthält 5 % Ibuprofen.

**Beispiel 8**

```
 5,00 g Ibuprofen
35,00 g Dimethylsulfoxid
15,50 g Polyoxyethylen-polyoxypropylen-copolymer 12500
        (Pluronic® F 127, Wyandotte Chemicals Corp.)
44,50 g Wasser
```

werden wie in Beispiel 4 beschrieben verarbeitet. Das Gel enthält 5 % Ibuprofen.

**Beispiel 9**

```
 5,00 g Ibuprofen
14,00 g Isopropanol
10,00 g Dimethylisosorbid
30,00 g Polyoxyethylen-polyoxypropylen-copolymer 6500
        (Pluronic® F 105, Wyandotte Chemicals Corp.)
41,00 g Wasser
```

werden wie in Beispiel 4 beschrieben verarbeitet. Das Gel enthält 5 % Ibuprofen.

**Beispiel 10**

```
 5,00 g Ibuprofen
18,46 g Ethanol
11,54 g 1,2-Propandiol
15,50 g Polyoxyethylen-polyoxypropylen-copolymer 12500
        (Pluronic® F 127, Wyandotte Chemicals Corp.)
49,50 g Wasser
```

werden wie in Beispiel 4 beschrieben verarbeitet. Das Gel enthält 5 % Ibuprofen.

**Beispiel 11**

```
 5,00 g Ibuprofen
10,00 g Ethanol
18,00 g Isosorbid
15,50 g Polyoxyethylen-polyoxypropylen-copolymer 12500
        (Pluronic® F 127, Wyandotte Chemicals Corp.)
51,50 g Wasser
```

werden wie in Beispiel 4 beschrieben verarbeitet. Das Gel enthält 5 % Ibuprofen.

**Beispiel 12**

```
 5,00 g Flurbiprofen
17,00 g Isopropanol
10,00 g Dimethylisosorbid
16,00 g Polyoxyethylen-polyoxypropylen-copolymer 12500
        (Pluronic® F 127, Wyandotte Chemicals Corp.)
52,00 g Wasser
```

werden wie in Beispiel 4 beschrieben verarbeitet. Das Gel enthält 5 % Flurbiprofen.

**Beispiel 13**

```
 4,00 g Ketoprofen
15,00 g Isopropanol
 7,50 g Dimethylisosorbid
23,50 g Polyoxyethylen-polyoxypropylen-copolymer 12500
        (Pluronic® F 127, Wyandotte Chemicals Corp.)
50,00 g Wasser
```

werden wie in Beispiel 4 beschrieben verarbeitet. Das Gel enthält 4 % Ketoprofen.

**Beispiel 14**

```
 4,00 g Naproxen
12,00 g Isopropanol
30,00 g Dimethylsulfoxid
22,00 g Polyoxyethylen-polyoxypropylen-copolymer 12500
        (Pluronic® F 127, Wyandotte Chemicals Corp.)
32,00 g Wasser
```

werden wie in Beispiel 4 beschrieben verarbeitet. Das Gel enthält 4 % Naproxen.

**Beispiel 15**

```
 4,00 g Naproxen
10,00 g Ethanol
30,00 g Dimethylsulfoxid
24,00 g Polyoxyethylen-polyoxypropylen-copolymer 12500
        (Pluronic® F 127, Wyandotte Chemicals Corp.)
32,00 g Wasser
```

werden wie in Beispiel 4 beschrieben verarbeitet. Das Gel enthält 4 % Naproxen.

**Beispiel 16**

```
 5,00 g Iboprofen
19,00 g Isopropanol
10,00 g Dimethylisosorbid
10,00 g Polyoxyethylen-polyoxypropylen-copolymer 12500
        (Pluronic® F 127, Wyandotte Chemicals Corp.)
56,00 g Wasser
```

werden unter Rühren auf 50 °C erwärmt und solange gerührt, bis eine klare Lösung entstanden ist. Anschliessend wird bei laufendem Rührwerk auf Raumtemperatur abgekühlt.
Die Lotion enthält 5 % Ibuprofen.

**Beispiel 17**

```
15,00 g Ibuprofen
35,00 g Isopropanol
15,00 g Dimethylisosorbid
25,00 g Polyoxyethylen-polyoxypropylen-copolymer 1900
        (Pluronic® L 35, Wyandotte Chemicals Corp.)
10,00 g Wasser
```

werden bei 40 °C wie in Beispiel 16 beschrieben verarbeitet.
Die Lotion enthält 15 % Ibuprofen.

**Beispiel 18**

```
15,00 g Ibuprofen
30,00 g Isopropanol
15,00 g Dimethylisosorbid
25,00 g Polyoxyethylen-polyoxypropylen-copolymer 1900
        (Pluronic® L 35, Wyandotte Chemicals Corp.)
15,00 g Wasser
```

werden bei 40 °C wie in Beispiel 16 beschrieben verarbeitet.
Die Lotion enthält 15 % Ibuprofen.

**Beispiel 19**

```
 5,00 g Ketoprofen
17,00 g Isopropanol
10,00 g Dimethylisosorbid
16,00 g Polyoxyethylen-polyoxypropylen-copolymer 12500
        (Pluronic® F 127, Wyandotte Chemicals Corp.)
52,00 g Wasser
```

werden wie in Beispiel 16 beschrieben verarbeitet.
Die Lotion enthält 5 % Ketoprofen.

**Beispiel 20**

```
 4,00 g Naproxen
17,00 g Isopropanol
15,00 g Dimethylisosorbid
30,00 g Polyoxyethylen-polyoxypropylen-copolymer 12500
        (Pluronic® F 127, Wyandotte Chemicals Corp.)
34,00 g Wasser
```

werden wie in Beispiel 16 beschrieben verarbeitet.
Die Lotion enthält 4 % Naproxen.

**Beispiel 21**

```
10,000 g Ibuprofen
18,000 g Isopropanol
10,000 g Dimethylisosorbid
 2,000 g Polyoxypropylen-(15)-stearylether
19,000 g Polyoxyethylen-polyoxypropylen-copolymer 12500
         (Pluronic® F 127, Wyandotte Chemicals Corp.)
40,925 g Wasser
 0,025 g Lavendelöl
 0,050 g Neroliöl
```

werden wie in Beispiel 1 beschrieben verarbeitet. Das Gel enthält 10% Ibuprofen.

**Beispiel 22**

```
 5,00 g Ibuprofen
16,00 g Isopropanol
10,00 g Dimethylisosorbid
 1,00 g Polyoxyethylen-(4)-laurylether
16,00 g Polyoxyethylen-polyoxypropylen-copolymer 12500
        (Pluronic® F 127, Wyandotte Chemicals Corp.)
52,00 g Wasser
```

werden wie in Beispiel 4 beschrieben verarbeitet. Das Gel enthält 5% Ibuprofen.

**Beispiel 23**

```
0,400 kg Ibuprofen
1,200 kg Isopropanol
0,800 kg Dimethylisosorbid
1,280 kg Polyoxyethylen-polyoxypropylen-copolymer 12500
         (Pluronic® F 127, Wyandotte Chemicals Corp.)
4,234 kg Wasser
```

werden 10 Minuten lang intensiv gemischt. Die Mischung wird anschließend unter Rühren und leichtem Druck (0,3 bar) auf 85°C erhitzt und 15 Minuten bei dieser Temperatur belassen. Danach wird auf 65°C abgekühlt und mit Benzylnicotinat (0,080 kg) und Aromastoffen (2 g Lavendelöl und 4 g Neroliöl) versetzt. Die Lösung wird bei laufendem Rührwerk auf Raumtemperatur abgekühlt. Während der Abkühlphase geliert die Lösung ab einer Temperatur von ca. 45°C zu einem klaren Gel. Das Gel enthält 5% Ibuprofen.

**Beispiel 24**

```
 5,00 g Ibuprofen

16,00 g Isopropanol

10,00 g Dimethylisosorbid

16,00 g Polyoxyethylen-polyoxypropylen-copolymer 12500
        (Pluronic® F 127, Wyandotte Chemicals Corp.)

52,00 g Wasser
```

werden 10 Minuten intensiv gemischt. Die Mischung wird anschließend unter Rühren und leichtem Druck (0,3 bar) auf 85°C erhitzt und 15 Minuten bei dieser Temperatur belassen. Danach wird auf 65°C abgekühlt, mit Methylsalicylat (1,0 g) versetzt und wie in Beispiel 23 beschrieben weiterbehandelt. Das Gel enthält 5% Ibuprofen.

Die erfindungsgemäßen Zubereitungen werden je nach Art der zu behandel nden Krankheit appliziert. Vorzugsweise wird das entzündete bzw. schmerzhafte Gebiet 3 bis 6 mal täglich mit einem 5 bis 10 cm langen Strang Gel oder einer entsprechenden Menge flüssiger Einreibung eingerieben, so daß etwa 100 bis 500 mg Arylpropionsäure aufgebracht werden. Als Indikationen kommen in Betracht degenerative und entzündliche Gelenkerkrankungen, Weichteilrheumatismus, Lumbago, Myogelosen und traumatische Zustände nach Sportverletzungen und Unfällen.

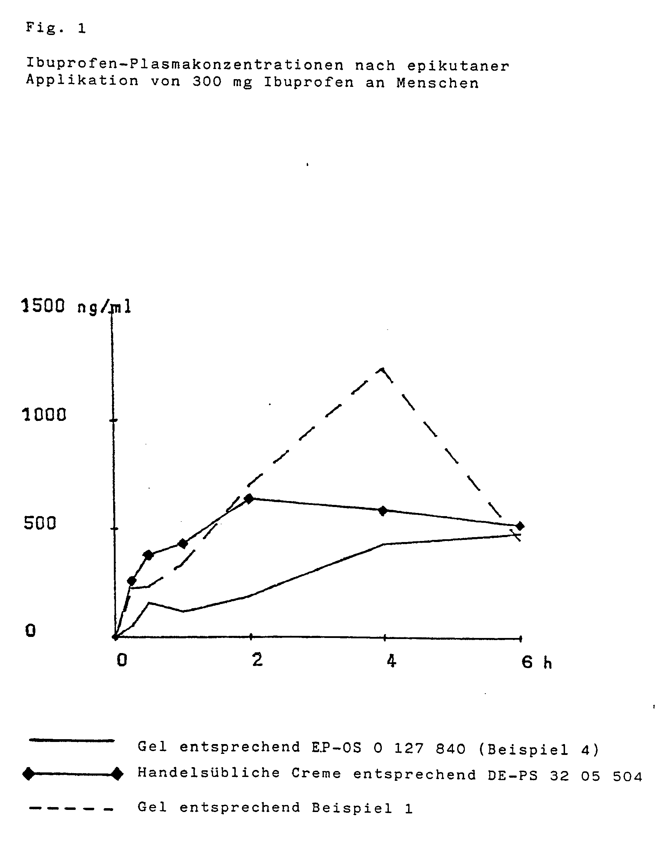

Die Bioverfügbarkeit von Ibuprofen aus einem erfindungsgemäßen Gel (Beispiel 1) wurde im Vergleich zu einer handelsüblichen Creme gemäß DE-PS 32 05 504 und einem Gel gemäß EP-OS 0 127 840 (Beispiel 4) an acht Probanden geprüft. Dazu wurde auf der oberen Rückenpartie der Versuchspersonen ein 20 x 20 cm großes Hautareal farblich markiert. Auf dieser Fläche wurde die Menge der Versuchspräparate aufgetragen, die 300 mg Ibuprofen entspricht. Die Präparate wurden für die Dauer von einer Minute von einer Hilfsperson eingerieben, deren Hand mit einem Gummihandschuh geschützt war. Vor dem Einreiben sowie nach 0,25, 0,5, 1, 2, 4 und 6 Stunden erfolgten Blutabnahmen. Aus dem Blut wurde durch Zentrifugieren unter Zusatz von Ethylendiamintetraessigsäure Plasma gewonnen, in dem über HPLC-Trennung mit einem elektrochemischen Detektor das Ibuprofen bestimmt wurde. Die Mittelwerte der Bestimmungen sind in Figur 1 als Kurve dargestellt.

Unter der Bioverfügbarkeit eines Arzneistoffes aus einer Zubereitung versteht man die Geschwindigkeit und das Ausmaß, mit denen dieser in die Blutbahn gelangt (E. Mutschler, Arzneimittel-Wirkungen, Wissenschaftl. Verlagsgesellschaft mbH, Stuttgart 1981).

Nach F.H. Dost (Grundlagen der Pharmakokinetik, Georg Thieme Verlag, Stuttgart 1968) entspricht die Fläche, welche die Blutspiegelkurve mit der Zeitachse umschließt (AUC = area under the curve) der Substanzmenge im Organismus. Daraus ergibt sich die Möglichkeit, die Bioverfügbarkeit einer Substanz aus verschiedenen Darreichungsformen zu vergleichen. Aus den in Figur 1 dargestellten Plasmaspiegelverläufen wurden die AUC-Werte berechnet und die relative Bioverfügbarkeit des Ibuprofens ermittelt (Tabelle 1).

11

## T a b e l l e   1

| | AUC-Werte h x ng/ml | relative Bio- verfügbarkeit | |
|---|---|---|---|
| Gel entsprechend Beispiel 1 | 4388,8 | 2,45 | 1,38 |
| Handelsübliche Cre- me entsprechend DE-PS 32 05 504 | 3190,0 | 1,78 | 1 |
| Gel entsprechend EP-OS 0 127 840 (Beispiel 4) | 1788,2 | 1 | 0,56 |

Tabelle 1 zeigt, daß mit der erfindungsgemäßen Gelpräparation die Bioverfügbarkeit des Wirkstoffes gegenüber einem vorbeschriebenen Gelpräparat bzw. einer handelsüblichen Creme um 145 % bzw 38 % verbessert wurde.

Darüber hinaus ist, wie aus dem Vergleich des Anstiegs der Plasmaspiegelkurven der beiden Gelpräparate ersichtlich ist, bei dem erfindungsgemäßen Gel eine schnellere Anflutung des Wirkstoffes als bei dem vorbekannten Gel gegeben. Mit der schnelleren und höheren Freisetzung und Bioverfügbarkeit von Arylpropionsäuren aus den erfindungsgemäßen Gelpräparaten stehen somit besser und schneller wirksame transdermal applizierbare, entzündungshemmend wirkende Arzneimittel als bisher zur Verfügung.

**Ansprüche**

1. Transdermal resorbierbare, wasserhaltige Zubereitungen von Arylpropionsäuren, dadurch gekennzeichnet, daß sie neben 1 bis 15 Gew.-% Wirkstoff, 10 bis 40 Gew.-% Polyoxyethylen-polyoxypropylen-copolymere, 10 bis 50 Gew.-% eines oder mehrerer physiologisch verträglicher organischer Lösungsmittel, gegebenenfalls bis zu 1 Gew.-% Konservierungsstoffe, Farbstoffe und/oder Parfüme und mindestens 10 Gew.-% Wasser enthalten.

2. Zubereitungen gemäß Anspruch 1, dadurch gekennzeichnet, daß sie 1 bis 10 Gew.-% Wirkstoff, 15 bis 32 Gew.-% Polyoxyethylen-polyoxypropylen-copolymere, 17 bis 44 Gew.-% eines oder mehrerer physiologisch verträglicher organischer Lösungsmittel enthalten.

3. Zubereitungen gemäß Anspruch 1 oder 2, dadurch gekennzeichnet, daß die Polyoxyethylen-polyoxypropylen-copolymere relative Molmassen von 1000 bis 13000 aufweisen.

4. Zubereitungen gemäß Anspruch 1 oder 2, dadurch gekennzeichnet, daß die Polyoxyethylen-polyoxypropylen-copolymere relative Molmassen von 6000 bis 13000 aufweisen.

5. Zubereitungen gemäß Anspruch 4, dadurch gekennzeichnet, daß sie in Form von Gelen vorliegen.

6. Zubereitungen gemäß einem der Ansprüche 1 bis 5, dadurch gekennzeichnet, daß als physiologisch verträgliche, organische Lösungsmittel verwendet werden Dimethylsulfoxid oder ein-bis dreiwertige, geradkettige, verzweigte oder cyclische Alkohole mit 2 bis 6 Kohlenstoffatomen, in deren Kohlenstoffgerüst auch bis zu 2 Sauerstoffatome eingebaut sein können, oder Polyoxyalkylenfettalkoholether oder Gemische derselben.

7. Zubereitungen gemäß einem der Ansprüche 1 bis 6, dadurch gekennzeichnet, daß zusätzlich bis zu 2 Gew.-% durchblutungsfördernde bzw. wärmende Stoffe enthalten sind.

8. Zubereitung gemäß Anspruch 7, dadurch gekennzeichnet, daß der durchblutungsfördernde bzw. wärmende Stoff Benzylnicotinat ist.

9. Zubereitung gemäß Anspruch 7, dadurch gekennzeichnet, daß der durchblutungsfördernde bzw. wärmende Stoff ein Salicylat ist.

10. Zubereitungen gemäß Anspruch 9, dadurch gekennzeichnet, daß als Lösungsmittel verwendet werden Ethanol, Isopropanol, 1,2-Propandiol, Glycerin, Isosorbid, Dimethylisosorbid, Polyoxyethylen-(4)-laurylether, Polyoxypropylen-(15)-stearylether oder Gemische derselben.

11. Verfahren zur Herstellung von transdermal resorbierbaren, wasserhaltigen Zubereitungen von Arylpropionsäurederivaten,
    dadurch gekennzeichnet, daß 1 bis 15 Gew.-% Wirkstoff, 10 bis 40 Gew.-% Polyoxyethylen-polyoxypropylen-copolymere, 10 bis 50 Gew.-% eines oder mehrerer physiologisch verträglicher organischer Lösungsmittel, gegebenenfalls bis zu 1 Gew.-% Konservierungsstoffe, Farbstoffe und/oder Parfüme und mindestens 10 Gew.-% Wasser, gegebenenfalls unter Überdruck unter Rühren auf 40 bis 90 °C erwärmt und wieder abgekühlt werden.

12. Verfahren gemäß Anspruch 11 zur Herstellung von Gelen, dadurch gekennzeichnet, daß 1 bis 10 Gew.-% Wirkstoff, 15 bis 32 Gew.-% Polyoxyethylen-polyoxypropylen-copolymere mit einer relativen Molmasse von mindestens 6000, 17 bis 44 Gew.-% eines oder mehrerer physiologisch verträglicher organischer Lösungsmittel auf 80 bis 90 °C erwärmt und wieder abgekühlt werden, worauf gegebenenfalls Konservierungsstoffe, Farbstoffe und/oder Parfüme zugegeben werden.

13. Verfahren gemäß Anspruch 11 oder 12, dadurch gekennzeichnet, daß als physiologisch verträgliche organische Lösungsmittel verwendet werden Dimethylsulfoxid oder ein- bis dreiwertige, geradkettige, verzweigte oder cyclische Alkohole mit 2 bis 6 Kohlenstoffatomen, in deren Kohlenstoffgerüst auch bis zu 2 Sauerstoffatome eingebaut sein können, oder Polyoxyalkylen-fettalkoholether oder Gemische derselben.

14. Verfahren gemäß Anspruch 13, dadurch gekennzeichnet, daß als Lösungsmittel verwendet werden Ethanol, Isopropanol, 1,2-Propandiol, Glycerin, Isosorbid, Dimethylisosorbid, Polyoxyethylen-(4)-laurylether, Polyoxypropylen-(15)-stearylether oder Gemische derselben.

15. Verfahren gemäß Ansprüchen 11 bis 14, dadurch gekennzeichnet, daß zusätzlich bis zu 2 Gew.-% durchblutungsfördernde bzw. wärmende Stoffe zugegeben werden.

16. Verfahren gemäß Anspruch 15, dadurch gekennzeichnet, daß der durchblutungsfördernde bzw. wärmende Stoff Benzylnicotinat ist.

17. Verfahren gemäß Anspruch 15, dadurch gekennzeichnet, daß der durchblutungsfördernde bzw. wärmende Stoff ein Salicylat ist.

## Claims

1. Transdermally resorbable water-containing preparations of arylpropionic acids, characterized in that they contain, in addition to 1 to 15% by weight of the active ingredient, from 10 to 40% by weight of polyoxyethylene polyoxypropylene copolymers, from 10 to 50% by weight of one or more physiologically acceptable organic solvents, optionally up to 1% by weight of preservatives, colorants and/or perfumes, and at least 10% by weight of water.

2. Preparations according to claim 1, characterized in that they contain from 1 to 10% by weight of the active ingredient, from 15 to 32% by weight of polyoxyethylene polyoxypropylene copolymers, from 17 to 44% by weight of one or more physiologically acceptable organic solvents.

3. Preparations according to claims 1 or 2, characterized in that the polyoxyethylene polyoxypropylene copolymers have relative molar masses of from 1,000 to 13,000.

4. Preparations according to claims 1 or 2, characterized in that the polyoxyethylene polyoxypropylene copolymers have relative molar masses of from 6,000 to 13,000.

5. Preparations according to claim 4, characterized in that they are in the form of gels.

6. Preparations according to any one of claims 1 to 5, characterized in that, as the physiologically acceptable organic solvents, there are used dimethyl sulfoxide or mono- to trihydric straight-chain, branched or cyclic alcohols having from 2 to 6 carbon atoms in the carbon backbone of which also up to 2 oxygen atoms may have been incorporated, or polyoxyalkylene fatty alcohol ethers or mixtures thereof.

7. Preparations according to any one of claims 1 to 6, characterized in that they additionally contain up to 2% by weight of blood circulation-promoting or warming materials, respectively.

8. Preparations according to claim 7, characterized in that the blood circulation-promoting or warming material, respectively, is benzyl nicotinate.

9. Preparations according to claim 7, characterized in that the blood circulation-promoting or warming material, respectively, is a salicylate.

10. Preparations according to claim 9, characterized in that, as solvents, there are used ethanol, isopropanol, 1,2-propane diol, glycerol, isosorbide, dimethylisosorbide, polyoxyethylene-(4)-lauryl ether, polyoxypropylene-(15)-stearyl ether or mixtures thereof.

11. A process for making transdermally resorbable water-containing preparations of arylpropionic acids, characterized in that from 1 to 15% by weight of the active ingredient, from 10 to 40% by weight of polyoxyethylene polyoxypropylene copolymers, from 10 to 50% by weight of one or more physiologically acceptable organic solvents, optionally up to 1% by weight of preservatives, colorants and/or perfumes, and at least 10% by weight of water are heated with stirring, optionally under elevated pressure, at from 40° C to 90° C and then cooled again.

12. The process according to claim 11 for making gels, characterized in that from 1 to 10% by weight of the active ingredient, from 15 to 32% by weight of polyoxyethylene polyoxypropylene copolymers having a relative molar mass of at least 6,000, from 17 to 44% by weight of one or more physiologically acceptable organic solvents are heated at from 80° C to 90° C and then cooled again whereupon, optionally, the preservatives, colorants and/or perfumes are added.

13. The process according to claims 11 or 12, characterized in that, as the physiologically acceptable organic solvents, there are used dimethyl sulfoxide or mono- to trihydric straight-chain, branched or cyclic alcohols having from 2 to 6 carbon atoms in the carbon backbone of which also up to 2 oxygen atoms may have been incorporated, or polyoxyalkylene fatty alcohol ethers or mixtures thereof.

14. The process according to claim 13, characterized in that, as solvents there are used ethanol, isopropanol, 1,2-propane diol, glycerol, isosorbide, dimethylisosorbide, polyoxyethylene-(4)-lauryl ether, polyoxypropylene-(15)-stearyl ether or mixtures thereof.

15. Process according to any one of claims 11 to 14, characterized in that there are further added up to 2% by weight of blood circulation-promoting or warming materials, respectively.

16. Process according to claim 15, characterized in that the blood circulation-promoting or warming material, respectively, is benzyl nicotinate.

EP 0 215 423 B1

**17.** Process according to claim 15, characterized in that the blood circulation-promoting or warming material, respectively, is a salicylate.

**Revendications**

1. Préparations aqueuses résorbables par voie transdermale d'acides arylpropioniques, caractérisées en ce qu'elles contiennent, à côté de 1 à 15 % en poids de matière active, 10 à 40 % en poids de copolymères polyoxyéthylène-polyoxypropylène, 10 à 50 % en poids d'un ou plusieurs solvants organiques physiologiquement compatibles, éventuellement jusqu'à 1 % en poids de conservateurs, colorants et/ou parfums, et au moins 10 % en poids d'eau.

2. Préparations selon la revendication 1, caractérisée en ce qu'elles contiennent 1 à 10 % en poids de matières actives, 15 à 32 % en poids de copolymères polyoxyéthylène-polyoxypropylène, 17 à 44 % en poids d'un ou plusieurs solvants organiques physiologiquement compatible.

3. Préparations selon la revendication 1 ou 2, caractérisée en ce que les copolymères polyoxyéthylène-polyoxypropylène ont une masse molaire relative de 1000 à 13000.

4. Préparations selon la revendication 1 ou 2, caractérisée en ce que les copolymères polyoxyéthylène-polyoxypropylène ont une masse molaire relative de 6000 à 13000.

5. Préparations selon la revendication 4, caractérisée en ce qu'elles se présentent sous forme de gels.

6. Préparations selon l'une des revendications 1 à 5, caractérisées en ce qu'on utilise, comme solvants organiques physiologiquement compatibles, du diméthylsulfoxyde ou des alcools mono à trivalents, à chaîne droite, ramifiée ou cyclique, contenant 2 à 6 atomes de carbone, dans lequel le squelette carboné peut contenir aussi jusqu'à deux atomes d'oxygène, ou un polyoxyalkylène-éther d'alcool gras, ou des mélanges de ces substances.

7. Préparations selon l'une des revendications 1 à 6, caractérisées en ce qu'elles contiennent en plus jusqu'à 2 % en poids de substances accélérant la circulation du sang ou produisant un échauffement.

8. Préparations selon la revendication 7, caractérisées en ce que la substance accélérant la circulation du sang ou produisant un échauffement est le nicotinate de benzyle.

9. Préparations selon la revendication 7, caractérisées en ce que la substance accélérant la circulation du sang ou produisant un échauffement est un salicylate.

10. Préparations selon la revendication 9, caractérisées en ce qu'on utilise comme solvant, de l'éthanol, de l'isopropanol, du 1,2-propanediol, de la glycérine, de l'isosorbide, du diméthyl-isosorbide, du polyoxyéthylène-(4)- éther de lauryle, du polyoxypropylène-(15)-éther de stéaryle ou des mélanges de ces substances.

11. Procédé d'obtention de préparations aqueuses résorbables par voie transdermale d'acides arylpropioniques, caractérisé en ce qu'on chauffe à 40 à 90°C sous pression et en agitant, 1 à 15 % en poids de matière active, 10 à 40 % en poids de copolymères polyoxyéthylène-polyoxypropylène, 10 à 50 % en poids d'un ou plusieurs solvants organiques physiologiquement compatibles, éventuellement jusqu'à 1 % en poids de conservateurs, colorants et/ou parfums, et au moins 10 % en poids d'eau, et on refroidit ensuite.

12. Procédé selon la revendication 11 pour l'obtention de gels, caractérisé en ce qu'on chauffe à 80 à 90°, et on refroidit ensuite, 1 à 10 % en poids de matière active, 15 à 32 % en poids de copolymères polyoxyéthylène-polyoxypropylène ayant une masse molaire relative d'au moins 6000 et 17 à 44 % en poids d'un ou plusieurs solvants organiques physiologiquement compatibles, après quoi on ajoute éventuellement les conservateurs, colorants et/ou parfums.

13. Procédé selon la revendication 11 ou 12, caractérisées en ce qu'on utilise, comme solvants organiques

15

physiologiquement compatibles, du diméthylsulfoxyde ou des alcools mono à trivalents, à chaîne droite, ramifiée ou cyclique, contenant 2 à 6 atomes de carbone, dans lequel le squelette carboné peut contenir aussi jusqu'à deux atomes d'oxygène, ou un polyoxyalkylène-éther d'alcool gras, ou des mélanges de ces substances.

14. Procédé selon la revendication 13, caractérisé en ce qu'on utilise comme solvant, de l'éthanol, de l'isopropanol, du 1,2-propanediol, de la glycérine, de l'isosorbide, du diméthyl-isosorbide, du polyoxyéthylène-(4)-éther de lauryle, du polyoxypropylène-(15)-éther de stéaryle ou des mélanges de ces substances.

15. Procédé selon les revendications 1, 11 à 14, caractérisé en ce qu'on ajoute en plus jusqu'à 2 % de substances accélérant la circulation du sang ou produisant un échauffement.

16. Procédé selon la revendication 15, caractérisé en ce que la substance accélérant la circulation du sang ou produisant un échauffement est le nicotinate de benzyle.

17. Procédé selon la revendication 15, caractérisé en ce que la substance accélérant la circulation de sang ou provoquant un échauffement est un salicylate.

Fig. 1

Ibuprofen-Plasmakonzentrationen nach epikutaner
Applikation von 300 mg Ibuprofen an Menschen

—————————— Gel entsprechend EP-OS 0 127 840 (Beispiel 4)

◆——————◆ Handelsübliche Creme entsprechend DE-PS 32 05 504

– – – – – Gel entsprechend Beispiel 1